# EUROPEAN PATENT APPLICATION

(11) **EP 2 660 330 A1**
(43) Date of publication of application: **06.11.2013**
(21) Application number: 11852599.7
(22) Date of filing: 31.10.2011
(51) Int. Cl.: C12Q 1/37, C12M 1/34

(54) **ENVIRONMENTAL BIOLOGICAL ALLERGEN MEASUREMENT METHOD, AND SIMPLE KIT FOR MEASURING BIOLOGICAL ALLERGEN**

(30) Priority: 28.12.2010 JP 2010292593
(71) Applicant: Sunstar Engineering Inc., Osaka 569-1195 (JP)
(72) Inventor: ISHII Miwa, Takatsuki-shi Osaka 569-1195 (JP); IDEGUCHI Masumi, Takatsuki-shi Osaka 569-1195 (JP); OKA Toru, Takatsuki-shi Osaka 569-1195 (JP)
(74) Representative: Vossius & Partner
(86) International application number: PCT/JP2011/075067
(87) International publication number: WO 2012/090582

(57) **Abstract**

It is an object of this invention to provide an environmental biological allergen measurement method capable of simple and low-cost measurement of the amount of an environmental biological allergen without using anti-allergen antibodies or a special substrate, and to provide a simple biological allergen quantification kit for carrying out the method. The object can be achieved by a biological allergen measurement method characterized in that the amount of an environmental biological allergen is measured by bringing a test substance into contact with a water-soluble gel or aqueous solution containing a substrate of the protease of the environmental biological allergen, and by quantifying the physical change of the water-soluble gel or the aqueous solution caused by the effect of the protease contained in the test substance on the substrate.

## Description

### Technical Field

The present invention relates to an environmental biological allergen measurement method, and a simple kit for measuring biological allergen.

### Background Art

It is known that, in an environment, such as an indoor environment, mites, pollen, and the like exist, and the mites, pollen, and the like become an allergen to cause allergy diseases, such as atopic dermatitis. To such allergy diseases, it is important to avoid the contact with the allergen as much as possible to reduce an allergic reaction. Therefore, various measures for removing the allergen from the indoor environment and preventing invasion of the allergen into the indoor environment have been proposed, for example. However, in order to demonstrate the effect capable of avoiding the contact with the allergen, it is necessary to confirm how much the allergen exists in the indoor environment.

Heretofore, the measurement of the allergen has been performed by an immunoassay using an antibody in which the allergen to be measured is a homologous antigen (Patent Documents 1 to 5). However, the antibody for use in the immunoassay, particularly, a monoclonal antibody to be used in order to increase the measurement accuracy, is expensive and needs to pass through a complicated process. Therefore, a measurement method and a measurement kit capable of measuring the allergen in a simple and low cost manner are required.

As improvement measures therefore, some methods for measuring the allergen amount in an environment without using anti-allergen antibodies have been proposed in recent years (Patent Documents 6 and 7). These methods employ a measurement method focusing on the protease activity contained in the allergen and using a substrate whose specific bond is cut due to an enzyme reaction by the protease to cause a color change.
However, according to these methods, the measurement of the allergen is achieved in a simple manner to some extent as compared with the immunoassay but it is necessary to newly synthesize a special substrate to which a pigment which develops color as a result of reacting to the protease is bonded. Thus, the methods still have posed problems with the cost and the like.

### Citation List

### Patent Literatures

Patent Document 1: JP-A No. H09-87298
Patent Document 2: JP-A No. H05-207892
Patent Document 3: JP-A No. H11-14511
Patent Document 4: JP-A No. 2000-35428
Patent Document 5: JP-A No. H06-34518
Patent Document 6: JP-A No. 2006-345801
Patent Document 7: JP-A No. 2008-29337

### Summary of Invention

### Technical Problem

In view of the above-described problems, it is an object of the present invention to provide an environmental biological allergen measurement method capable of simple and low-cost measurement of the amount of an environmental biological allergen without using anti-allergen antibodies or a special substrate, and to provide a simple biological allergen quantification kit for carrying out the method.

### Solution to Problem

The present inventors have conducted extensive research, and, as a result, have found that, due to an effect of a water-soluble gel or aqueous solution containing a substrate of a protease of an environmental biological allergen on the substrate of the protease of the allergen, the physical properties of the water-soluble gel or the aqueous solution change, and the physical property change can be quantified. Thus, the present inventors have accomplished the present invention.
More specifically, the gist of the present invention is as follows.

A first aspect of the present invention relates to a biological allergen measurement method including bringing a test substance into contact with a water-soluble gel or aqueous solution containing a substrate of a protease of an environmental biological allergen, and then quantifying the physical property change of the water-soluble gel or the aqueous solution caused by the effect of the protease contained in the test substance on the substrate to thereby measure the amount of the environmental biological allergen.

In the present invention, the physical property change may be liquefaction of the water-soluble gel, and the amount of the environmental biological allergen may be measured by quantifying the weight or the volume of a portion generated by the liquefaction. In this case, it is preferable that the water-soluble gel contains protein which forms a gel at room temperature.

In the present invention, the physical property change may be a change in the pH of the water-soluble gel or the aqueous solution, and the amount of the environmental biological allergen may be measured by quantifying the pH change. In this case, it is preferable that the water-soluble gel or the aqueous solution contains protein whose pH changes due to the effect of the protease of the allergen. It is preferable that the water-soluble gel or the aqueous solution contains a pH indicator.

In the present invention, it is preferable that the substrate contains natural product-derived protein and/or protein hydrolyzate.

In the present invention, it is preferable that the test substance is collected from a floor, a wall, a window, a window frame, matting, bedding, furniture, dust particles, and house dust.

A second aspect of the present invention relates to a simple biological allergen measurement kit for use in the biological allergen measurement method, containing the water-soluble gel or the aqueous solution.

### Advantageous Effects of Invention

According to the present invention, since an anti-allergen antibody is not used or a color change or the like caused by a special substrate is not used as an index but a physical property change of a water-soluble gel or aqueous solution containing a substrate of a protease of an environmental biological allergen, the amount of the environmental biological allergen can be measured in a simple and low cost manner.

### Brief Description of the Drawings

Fig. 1 shows the relationship between the concentration of a protease (Der f 1) and the reduction amount (based on weight) of a water-soluble gel (gelatin gel) in Example 1.
Fig. 2 shows the relationship between the concentration of a protease (Der f 1) and the reduction amount (based on volume) of a water-soluble gel (gelatin gel) in Example 2.
Fig. 3(a) shows a color change of a pH test paper after adding dropwise each measurement sample in Example 3 and Fig. 3(b) shows a color change after adding dropwise each solution whose pH value is adjusted to a pH test paper.
Fig. 4(a) shows a color change of a pH test paper after adding dropwise each test solution (before allowed to stand still) of Example 4 and Fig. 4(b) shows a color change of a pH test paper after adding dropwise each test solution (after allowed to stand still for 3 days) of Example 4.
Fig. 5(a) shows a water-soluble gel immediately after (before allowed to stand still) adding dropwise each sample of Example 5 and Fig. 5(b) shows a portion near the upper portion of the water-soluble gel in Fig. 5(a) in an enlarged manner.
Fig. 6(a) shows a color change of the water-soluble gel after adding dropwise each sample of Example 5 (after allowed to stand still for 24 hours) and Fig. 6(b) shows a portion near the upper portion of the water-soluble gel in Fig. 6(a) in an enlarged manner.
Fig. 7(a) shows a color change of a gelatin gel after allowed to stand still for 24 hours when only a mite feed is used as a measurement sample in Example 6 and Fig. 7(b) shows a color change of a gelatin gel after allowed to stand still for 24 hours when a Dermatophagoides farinae culture medium is used as a measurement sample.

### Description of Embodiments

According to a biological allergen measurement method of the present invention, first, a test substance is brought into contact with a water-soluble gel or aqueous solution containing a substrate of a protease of an environmental biological allergen. Then, when the biological allergen is contained in the test substance, a protease contained in the allergen acts on the substrate of the protease contained in the water-soluble gel or the aqueous solution, and, as a result, the physical property change of the water-soluble gel or the aqueous solution is caused. Then, by quantifying the physical property change, the amount of the environmental biological allergen can be measured. The physical property change is determined by the amount (specific activity) of the protease reacting to the substrate. Therefore, by quantifying or semi-quantifying the physical property change, the amount (specific activity) of the protease, i.e., the allergen amount, can be measured, quantified, or semi-quantified.

In the invention, the "semi-quantification" refers to showing a relative allergen amount corresponding to the qualitatively or quantitatively shown physical property change although it cannot be said the relationship between the allergen amount and a specific amount of the physical property change is a uniquely-defined quantitative relationship. The "showing the relative allergen amount" means that a judgment such that the allergen amount in a test sample serving as a measurement target is large/small based on a standard value or a sample serving as a standard, for example, can be performed.

Specifically, the following examples can be mentioned. As one example, when the physical property change is liquefaction of an aqueous gel described later, the allergen amount is relatively shown, e.g., the allergen amount is "small" or "large", based on a predetermined standard value or a sample serving as a standard corresponding to a specific liquefied amount or a specific liquefied amount range, for example. As another example, when the physical property change is a pH change described later, the allergen amount is relatively shown, e.g., the allergen amount is "small" or "large", based on a predetermined standard value or a sample serving as a standard corresponding to a specific pH value or a specific pH value range, for example. When the pH change is made to correspond to a change in the color of a pH test paper, the allergen amount is similarly relatively shown corresponding to a specific color or a specific color range, for example. When the pH change is made to correspond to a change in the color of a pH reagent, the allergen amount is similarly relatively shown corresponding to the density of a changed color, for example.

The physical property change is not particularly limited and it is preferable to adopt liquefaction of the water-soluble gel or a change in the pH of the water-soluble gel or the aqueous solution from the viewpoint of ease of quantification. These physical property changes are caused by the fact that a protease contained in an allergen reacts to a substrate thereof, and then the substrate is decomposed or undergoes a reduction in the molecular weight, so that the gel is liquefied or a protease decomposition product of the substrate shows basicity.

As the physical property change, when adopting the liquefaction of the water-soluble gel, the amount of the environmental biological allergen can be measured by quantifying the weight or the volume of a portion generated by the liquefaction. The measurement methods are not particularly limited and mentioned are, for example, (i) The liquefied portion is collected, and then the weight thereof is measured with an electrobalance or the like, (ii) The liquefied portion is collected, and then the volume thereof is measured by a measuring cylinder or the like, (iii) The liquefied portion is removed, the weight of the remaining gel portion is measured, and then a difference with the weight before the physical property change measured beforehand is calculated, (iv) The liquefied portion is removed, the volume of the remaining gel portion is measured, and then a difference with the volume before the physical property change measured beforehand is calculated, and the like. However, the invention is not limited thereto.
For the allergen (protease) amount and the weight/volume described above, an analytical curve is created using a standard substance of the allergen to determine the correlation beforehand.

When adopting a change in the pH of the water-soluble gel (including a liquefied portion when liquefied as a result of a reaction to a protease) or the aqueous solution as the physical property change, the pH change can be quantified or semi-quantified by the following method.

In the case of the water-soluble gel, the pH of the liquefied portion can be measured with a pH test paper, a pH meter, a pH indicator, or the like, for example. Or, by encapsulating the water-soluble gel and covering an alkaline solution therein, the pH change can be made more noticeable.
In the case of the aqueous solution, the pH of the aqueous solution can be measured with a pH test paper, a pH meter, a pH indicator, or the like similarly as in the case of the water-soluble gel. Moreover, the pH can be measured by a color development, a change color, or the like of a pH indicator contained in the aqueous solution beforehand.
With respect to the allergen (protease) amount and the pH value or the color development based on the pH value, the allergen amount can be quantified or semi-quantified by determining the correlation beforehand using a standard substance.

The substrate of the protease of the environmental biological allergen is protein or a protein hydrolyzate and is not particularly limited insofar as a substance contains protein or a protein hydrolyzate which is the substrate and can be formed into a water-soluble gel or an aqueous solution. As the protein, natural product-derived protein can be used as it is. Substances obtained by isolating a specific component from a mixture of the natural product-derived protein and the like are included in the protein in the invention. As the protein hydrolyzate, polypeptide whose structure is equal to or higher than that of dipeptide obtained by decomposing the natural product-derived protein by an enzyme and polypeptide synthesized from amino acid can also be used.
Since the substrate specificity of the protease varies depending on the type of the allergen, a specific allergen can be detected by selecting and using a substrate to which a protease of the allergen to be measured reacts.
Thus, in the invention, it is not necessary to bond a specific pigment to protein and polypeptide as in Patent Documents 6 and 7, for example.

As a specific example of the protein, egg protein, soybean protein, wheat protein, rice protein, corn protein, milk serum protein, lactoglobulin, collagen, betaine, mutastein, ice nucleating protein, lactoferrin, gelatin, rennet casein, αs1-casein, β-casein, lysozyme, hemoglobin, myoglobin, prealbumin, avidin, monellin, miraculin, fibrous protein, mucin, lectin, prothrombin, plasma protein, serum protein, glycoprotein, and the like are mentioned and can be used singly or in combination of two or more kinds thereof.

As the protein which forms a gel at normal temperature constituting the water-soluble gel, gelatin is preferable from the viewpoint of cost, availability, and the like.

As the protein constituting the aqueous solution, serum protein, such as bovine serum albumin (BSA), and gelatin are preferable from the viewpoint of cost, availability, and the like.

The content of the protein serving as the substrate in the water-soluble gel or the aqueous solution may be changed as appropriate according to the amount of the allergen to be measured. The content is about 0.01 to 0.2 g/g-gel in the water-soluble gel and about 0.001 to 0.1 g/ml in the aqueous solution.

When adopting a change in the pH of the water-soluble gel or the aqueous solution as the physical property change, the water-soluble gel or the aqueous solution may also contain a pH indicator. Specifically, phenol red, bromothymol blue, litmus, neutral red, cresol red, phenolphthalein, p-naphthol phthalein, and the like are mentioned. These reagents may be used singly or in combination of two or more kinds thereof. When combining two or more kinds thereof, the color development range and the developed color of each reagent may be considered.
The content of the pH indicator may be changed as appropriate according to the type thereof and the amount of the allergen to be measured and is about 0.0005 to 0.5 mg/g-gel in the water-soluble gel and about 0.0005 to 0.5mg/ml in the aqueous solution, for example.

In the invention, the water-soluble gel or the aqueous solution may contain arbitrary additives in addition to the protein, a water-soluble solvent, and a pH indicator mentioned above. As such additives, an antiseptic, a protease inhibitor, a protein crosslinking agent (improvement of the hardness of the gel), and the like are mentioned.

Since the substrate specificity of the protease varies depending on the type of the allergen, a substrate to which the protease of the allergen to be measured reacts is selected and used. The protease inhibitor is known. By causing a protease inhibitor which inhibits the protease activity of a specific allergen to coexist with the substrate for use in the measurement, the protease activity of the specific allergen to be targeted can be selectively measured excluding the protease activity of the specific allergen. As an example of the protease inhibitor, p-metacrybenzoic acid, diisopropyl fluorophosphoric acid, tosyl phenylalanyl chloromethyl ketone, subtilisin inhibitor, leupeptin, antipain, pepstatin, epoxy succinic acid derivatives, aprotinin, bestatin, phenylmethylsulfonyl fluoride, EDTA, EGTA, benzamidine, and the like can be mentioned. By selecting a suitable substrate and causing an inhibitor to the protease of the allergen to be excluded from the measurement to coexist as required, the type of the allergen to be measured can be narrowed down.

With respect to the water-soluble gel or the aqueous solution to be used in the invention, the protein mentioned above and the pH indicator and the additives to be added as required are added to a water-soluble solvent to thereby prepare a mixed solution. The water-soluble gel is obtained by forming the mixed solution into a gel according to a usual method.
The water-soluble solvent is not particularly limited insofar as the activity of the protease is not inhibited, and water, a phosphate buffer, a physiological saline, and the like can be used.

The test substance is not particularly limited insofar as the environmental biological allergen having a protease can be collected and may be one collected from, for example, a floor, a wall, a window, a window frame, textiles such as matting (a carpet, a tatami, a mat, and the like), bedding (bedding, a blanket, a pillow, a mattress, and the like), furniture (a chair, a desk, and the like), and clothes, dust particles, house dust, and the like.
The biological allergen which can be contained in the test substance thus collected is pollen, mold, insect bodies, such as mites, insect-derived substances (excrement, dead bodies, fragments, and the like), or the like. The type of pollen, mold, mites, and the like is not specified. As one which functions as a protease derived from various types of mites (insect bodies, excrement, dead bodies, fragments), Der p/f 1, Der p/f 3, Der p/f 6, Der p 9, and the like derived from Dermatophagoides farinae and Dermatophagoides pteronyssinus are mentioned. The substances affect the protein such as the substrate to perform an enzyme effect.
As the collected test substance, the collected one may be used as it is or an extract of the test substance may also be used. The extract can be prepared with water, an aqueous buffer, a physiological saline, and the like.

A biological allergen simple quantification kit of the invention contains a water-soluble gel or aqueous solution containing a substrate of a protease of an environmental biological allergen as an indispensable constituent. The water-soluble gel or the aqueous solution is sealed in a predetermined container, and then the container is opened when inspecting to be brought into contact with a test substance.
As such a container, the form may be selected as appropriate according to the aspect of one to be sealed (a water-soluble gel or an aqueous solution), a contact method with the test substance, the aspect of the above-described physical change (e.g., a weight/volume change, a pH change, or the like).
As one example, when using a water-soluble gel, and quantifying the physical property change based on the volume change, the use of a stick-like transparent container allows simple quantification of the reduction amount of the water-soluble gel from the height thereof.

In order to collect the test substance, an instrument for collecting the test substance, such as an adhesion seal, a cotton bud, a filter, and a cloth fiber, may be selected as appropriate and attached to the kit of the invention considering the collection target.
When measuring the allergen amount based on the pH change, a pH test paper, a pH indicator, and the like may be added as required.

### Examples

Hereinafter, the invention is described in more detail with reference to Examples.

### (Example 1)

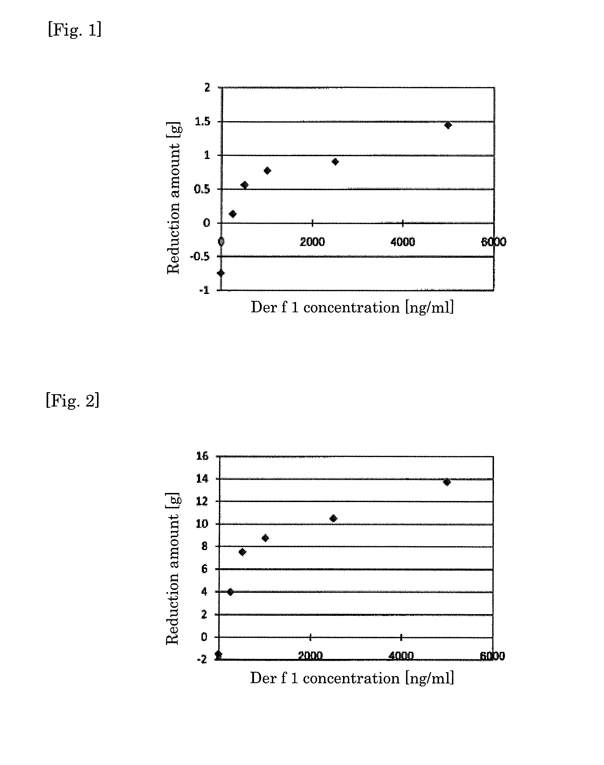

A water-soluble gel was obtained by using gelatin as a substrate of a protease, putting 8 ml of an aqueous 2% gelatin solution in a test tube (15 ml), and then forming a gel. 6 pieces of the test tubes containing the gelatin gel were prepared in total. The weight of each test tube was measured with an electrobalance.
As a test substance, a Dermatophagoides farinae crude extract (manufactured by ITEA, mite (Df) crude extract) was used. An allergen was Dermatophagoides farinae and a protease was Der f 1 mainly contained in Dermatophagoides farinae. The Dermatophagoides farinae crude extracts were diluted with water in such a manner that the Der f 1 concentration of each extract was 250, 500, 1000, 2500, and 5000 ng/ml to be used as measurement samples. As a measurement sample with a Der f 1 concentration of 0 ng/ml, water was used.
To each test tube containing the gelatin gel previously prepared, 1000 µl of the measurement sample of each concentration was added dropwise, and then the mixture was allowed to stand still at room temperature for 18 hours.
18 hours later, each test tube containing the gelatin gel was observed. Then, it was visually confirmed that the gelatin gel decreased and was liquefied with an increase in the concentration of the Der f 1. The liquefied portion was removed, the test tube was measured with an electrobalance, and then a difference from the weight measured beforehand was calculated as the reduction amount of the water-soluble (gelatin) gel. The results are shown in Table 1 and Fig. 1. Table 1 and Fig. 1 show that the reduction amount of the gel increases with an increase in the concentration of the Der f 1 which is the protease and the quantification of the allergen amount can be achieved by quantifying the physical property change (liquefaction) of the water-soluble gel based on the protease (Der f 1) as an index.
The concentration of the Der f 1 in the extract was quantified by an ELISA method (manufactured by Indoor) using an antigen-antibody effect.

**[Table 1]**

| Der f 1 concentration [ng/ml] | Reduction amount [g] |
|---|---|
| 0 | -0.74 |
| 250 | 0.137 |
| 500 | 0.562 |
| 1000 | 0.777 |
| 2500 | 0.907 |
| 5000 | 1.45 |

### (Example 2)

A water-soluble gel was obtained by using gelatin as a substrate of a protease, putting an aqueous 2% gelatin solution in a stick-like transparent container (15 mm in outer diameter and 150 mm in height) in such a manner that the liquid height was about 80 mm, and then forming a gel. 6 pieces of the transparent containers containing the gelatin gel were prepared in total. The height of the gelatin gel in the transparent container was measured.
As a test substance, the same Dermatophagoides farinae crude extract as that of Example 1 was used. The Dermatophagoides farinae crude extracts were diluted with a phosphate buffer in such a manner that the Der f 1 concentration of each extract was 250, 500, 1000, 2500, and 5000 ng/ml to be used as measurement samples. As a measurement sample with a Der f 1 concentration of 0 ng/ml, a phosphate buffer was used.
To each stick-like transparent container containing the gelatin gel previously prepared, 1000 µl of the measurement sample of each concentration was added dropwise, and then the mixture was allowed to stand still at room temperature for 18 hours.
18 hours later, each stick-like transparent container containing the gelatin gel was observed. Then, it was visually confirmed that the gelatin gel decreased and was liquefied with an increase in the concentration of the Der f 1. The liquefied portion was removed, the height of the gelatin gel was measured, and then a difference from the height of the gelatin gel measured beforehand was calculated as the reduction amount of the water-soluble (gelatin) gel. The reduction amount of the height is substantially equivalent to the reduction amount of the volume. The calculation results are shown in Table 2 and Fig. 2. Table 2 and Fig. 2 show that the reduction amount of the gel increases with an increase in the concentration of the Der f 1 which is the protease and the quantification of the allergen amount can be achieved by quantifying the physical property change (liquefaction) of the water-soluble gel based on the protease (Der f 1) as an index.

**[Table 2]**

| Der f 1 concentration [ng/ml] | Reduction amount [g] |
|---|---|
| 0 | -1.5 |
| 250 | 4 |
| 500 | 7.5 |
| 1000 | 8.75 |
| 2500 | 10.5 |
| 5000 | 13.75 |

### (Example 3)

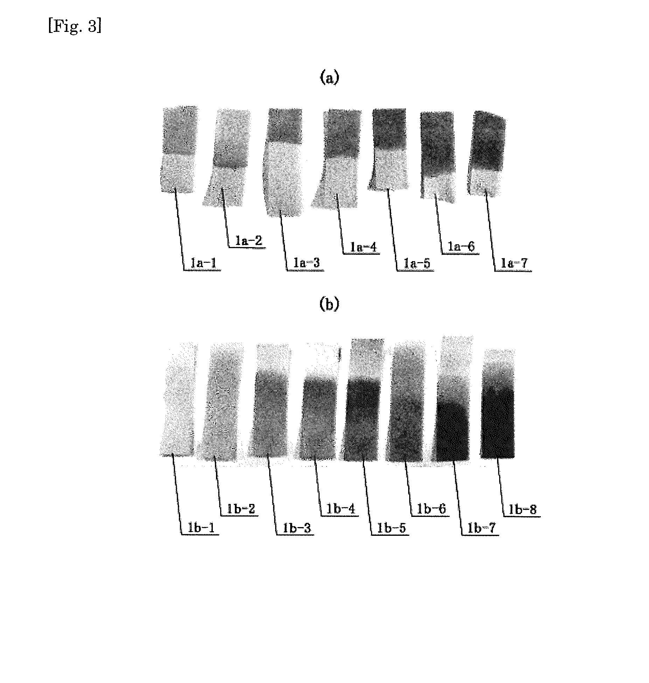

1000 µl of measurement samples with Der f 1 concentrations of 0 to 5000 ng/ml was added dropwise to each test tube containing a gelatin gel prepared beforehand, and then allowed to stand still at room temperature for 18 hours in the same manner as in Example 1. Another test tube to which 1000 µl of a measurement sample with a Der f 1 concentration of 5000 ng/ml was added dropwise was prepared. When the sample immediately after the dropwise addition was added dropwise to a pH test paper (manufactured by MACHEREY-NAGEL, Product name: Universal indicator paper, The test paper exhibits almost orange color when the liquid does not contact, exhibits almost yellowish green color when the liquid is neutral, and changes color in such a manner that green color becomes deep when the basicity becomes higher.), the pH test paper exhibited orange color.
18 hours later, it was visually confirmed that when each test tube containing the gelatin gel was observed, the gelatin gel was hardly liquefied when the concentration of the Der f 1 was 0 ng/ml but, in the sample containing the Der f 1, the gelatin gel decreased and was liquefied with an increase in the concentration. When each liquefied portion was added dropwise to the pH test paper, the sample immediately after the dropwise addition exhibited orange color as described above, but it was visually observed from a change in the color of the pH test paper that the basicity of the pH test paper became higher with an increase in the concentration of the Der f 1 (The color change and the pH value correspond to each other). The relationship between the color after the color of the pH test paper changed and the pH value was confirmed beforehand by adding dropwise a solution whose pH value was adjusted to the pH test paper. The results are shown in Figs. 3(a) and 3(b). Fig. 3(a) 1a-1 shows the color of the pH test paper of the sample immediately after adding dropwise the measurement sample with a Der f 1 concentration of 5000 ng/ml as described above, in which mainly the upper portion in the figure is a portion to which the sample was added dropwise. 1a-2 to 1a-7 show the color of the pH test paper of the liquefied portion after 18 hours passed after adding dropwise the samples with Der f 1 concentrations of 0, 250, 500, 1000, 2500, and 5000 ng/ml respectively. Fig. 3(b) shows the color after the solution whose pH value was adjusted was added dropwise to the pH test paper, in which mainly the lower portion in the figure is a portion to which the sample was added dropwise. The pH values of 1b-1 to 1b-8 are 5.0, 6.0, 6.5, 7.0, 7.5, 8.0, 8.2, and 8.5, respectively. From the contrast with Figs. 3(a) and 3(b), in 1a-1 and 1a-2, the color is approximate to the color of 1b-1 and 1b-2 and the pH is about 5.0 to 6.0. In 1a-3, the color is approximate to the color of 1b-4 and the pH is about 7.0. In 1a-4, the color is approximate to the color of 1b-5 and the pH is about 7.5. In 1a-5, the color is approximate to the color of 1b-6 and the pH is about 8.0. In 1a-6, the color is approximate to the color of 1b-7 and the pH is about 8.2. In 1a-7, the color is approximate to the color of 1b-7 and the pH is about 8.2.
Thus, immediately after adding dropwise the measurement sample with a Der f 1 concentration of 5000 ng/ml, the pH test paper exhibits orange color and is not basicity. However, when reacting with the gelatin gel for a given period of time to cause decomposition of the gelatin gel, the pH of the liquefied portion changes to basicity with the decomposition. After a given period of time passed, the pH which is the physical property of the portion where the gel-like gelatin was liquefied due to an enzyme reaction to the Der f 1 changed with an increase in the concentration of the Der f 1 which is the protease. Then, it is found that the measurement of the allergen amount can be achieved by quantifying the physical property change (pH change) of the water-soluble gel based on the protease (Der f 1) as an index.

### (Example 4)

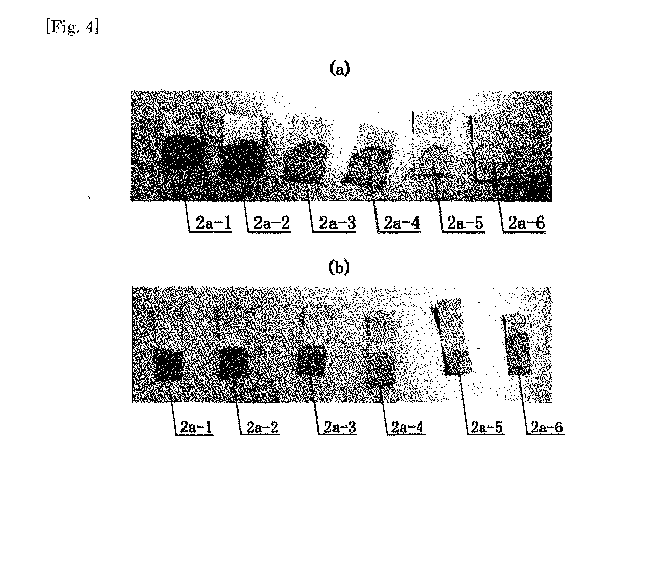

BSA (Bovine serum albumin) was used as a substrate of a protease, 1 ml of an aqueous 1.25% BSA solution was put in a glass container to form a water-soluble reactive substrate, and then 6 pieces of the containers were prepared in total. As a test substance, the same Dermatophagoides farinae crude extract as that of Example 1 was used. The Dermatophagoides farinae crude extracts were diluted with purified water in such a manner that the Der f 1 concentration of each extract was 5000, 2500, 1000, 500, and 250 ng/ml to be used as measurement samples (2a-1 to 2a-5). As a measurement sample (2a-6) with a Der f 1 concentration of 0 ng/ml, purified water was used.
500 µl of the measurement sample with each concentration was added dropwise to the glass container containing the aqueous BSA solution previously prepared to be used as test solutions. The pH of the test solutions was confirmed by a pH test paper (manufactured by MACHEREY-NAGEL, Product name: Universal indicator paper) (Fig. 4(a)). As shown in Fig. 4(a), 2a-1 to 2a-6 show the color change of the pH test paper immediately after adding dropwise the samples with Der f 1 concentrations of 5000, 2500, 1000, 500, 250, and 0 ng/ml, in which mainly the lower portion in the figure is a portion where the color changed. Since the color of the color-changed portion of 2a-1 almost coincides with 1b-4, the pH is 7.0.
Then, after allowed to stand still at room temperature for 3 days, the test solution was added dropwise to the pH test paper. Then, it was visually observed from the change in the color of the pH test paper that the basicity of the pH test paper became higher with an increase in the Der f 1 concentration (The color change and the pH value correspond to each other.) (Fig. 4(b)).
In Fig. 4(b), 2a-1 to 2a-6 show the color change of the pH test paper when the samples with Der f 1 concentrations of 5000, 2500, 1000, 500, 250, and 0 ng/ml were added dropwise respectively, in which mainly the lower portion in the figure is a portion where the color changed. Since the color of the color-changed portion of 2a-1 almost coincides with 1b-6, the pH is about 8.0. Since the color of the color-changed portion of 2a-2 almost coincides with 1b-6, the pH is about 8.0. Since the color of the color-changed portion of 2a-3 almost coincides with 1b-5, the pH is about 7.5. Since the color of the color-changed portion of 2a-4 almost coincides with 1b-4, the pH is about 7.0. Since the color of the color-changed portion of 2a-5 almost coincides with 1b-3, the pH is about 6.5. Since the color of the color-changed portion of 2a-6 almost coincides with 1b-3, the pH is about 6.5. The color change degree is strong as compared with the state before allowed to stand still in Fig. 4(a), and it is found that the color change dependent on the Der f 1 concentration, i.e., pH change, is observed.
Thus, it is found that the pH which is the physical property of the BSA solution was changed due to an enzyme reaction to the Der f 1 with an increase in the concentration of the Der f 1 which is the protease and the measurement of the allergen amount can be achieved by quantifying the physical property change (pH change) of the water-soluble substrate based on the protease (Der f 1) as an index.

### (Example 5)

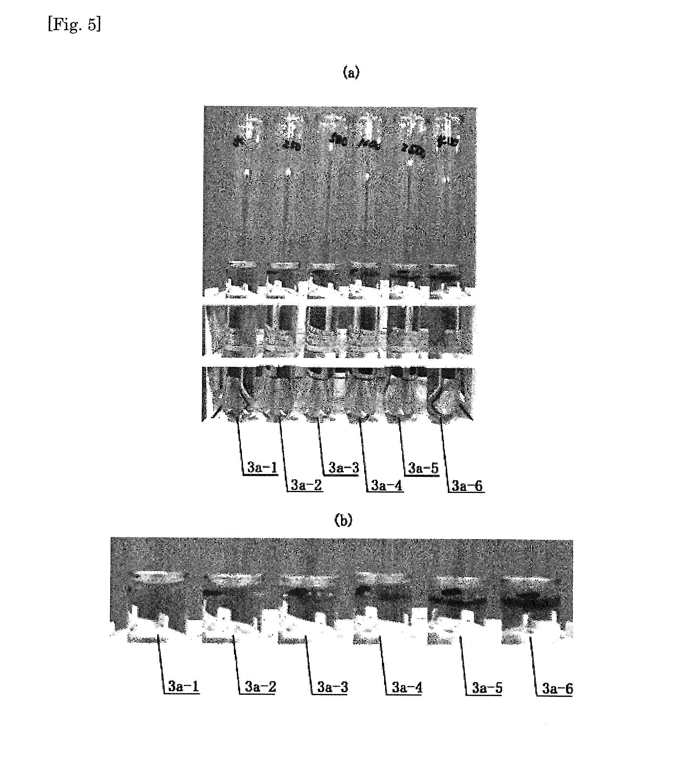

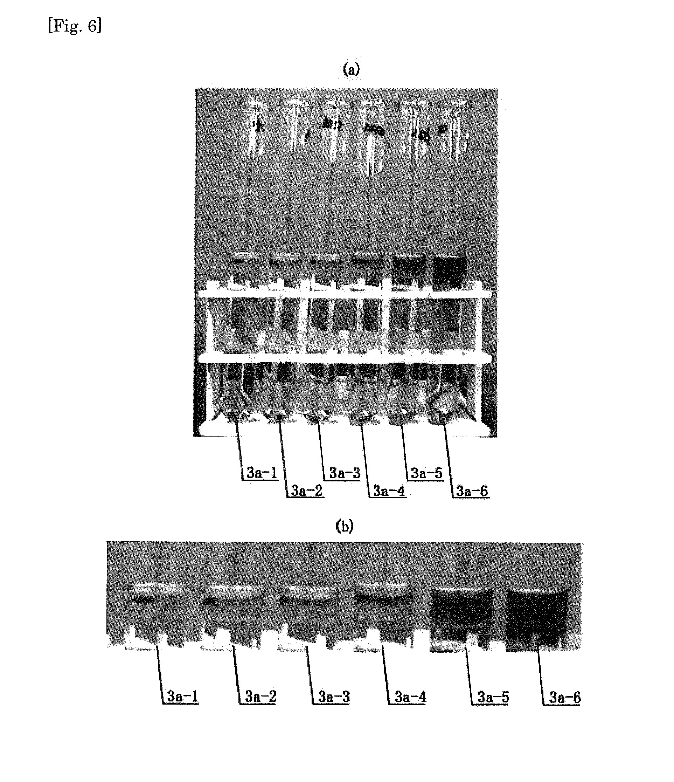

A water-soluble gel was obtained by using gelatin as a substrate of a protease, using phenol red as a pH indicator, adding phenol red (manufactured by SIGMA-ALDRICH, Product name: Phenol Red, A.C.S reagent, Color change range: pH 6.8 to pH 8.2, Color change from yellow to red) to an aqueous 2% gelatin solution, putting the mixture in a stick-like transparent container (15 mm in outer diameter and 150 mm in height) in such a manner that the liquid height was about 80 mm, and then forming a gel. 6 pieces of the transparent containers containing the gelatin gel were prepared in total.
As a test substance, the same Dermatophagoides farinae crude extract as that of Example 1 was used. The Dermatophagoides farinae crude extracts were diluted with purified water in such a manner that the Der f 1 concentration of each extract was 500, 1000, 2000, 5000, and 10000 ng/ml, and then 0.5 ml of the resultant solution was used as measurement samples 3a-2 to 3a-6. As a measurement sample (3a-1) with a Der f 1 concentration of 0 ng/ml, purified water was used.
500 µl of the measurement sample with each concentration was added dropwise to each stick-like transparent container containing the gelatin gel previously prepared. The color change immediately after the dropwise addition is shown in Figs. 5(a) and 5(b). Fig. 5(a) illustrates the entire stick-like transparent container. Fig. 5(b) shows a portion near the upper portion of the water-soluble gel in Fig. 5(a) in an enlarged manner. The water-soluble gel upper portion is in a state where the liquefied water-soluble gel and the sample were mixed.
After allowed to stand still at room temperature for 24 hours, each stick-like transparent container containing the gelatin gel was observed. Then, it was visually confirmed that the gelatin gel decreased and was liquefied with an increase in the Der f 1 concentration. Moreover, it was visually observed from a change in the color of the phenol red that the basicity of the liquefied portion became higher with an increase in the Der f 1 concentration (The color change and the pH value correspond to each other.). The color change in each sample at this time is shown in Figs. 6(a) and 6(b). Fig. 6(a) illustrates the entire stick-like transparent container. Fig. 6(b) shows a portion near the upper portion of the water-soluble gel in Fig. 6(a) in an enlarged manner. As shown in Fig. 6(b), it is found that the density of the color change to red color of the mixed portion of the liquefied water-soluble gel and the measurement sample and the height of the color-changed portion became high with an increase in the Der f concentration.
Thus, the pH which is the physical property of the portion where the gel-like gelatin was liquefied due to an enzyme reaction to the Der f 1 changed with an increase in the concentration of the Der f 1 which is the protease. It is considered that the measurement of the allergen amount can be achieved by quantifying or relatively showing the density of the color corresponding to the pH change and the height of the color-changed portion, quantitatively or relatively grasping the allergen amount corresponding thereto beforehand, and then quantifying or semi-quantifying the physical property change (pH change) of the water-soluble substrate based on the protease (Der f 1) as an index.

### (Example 6)

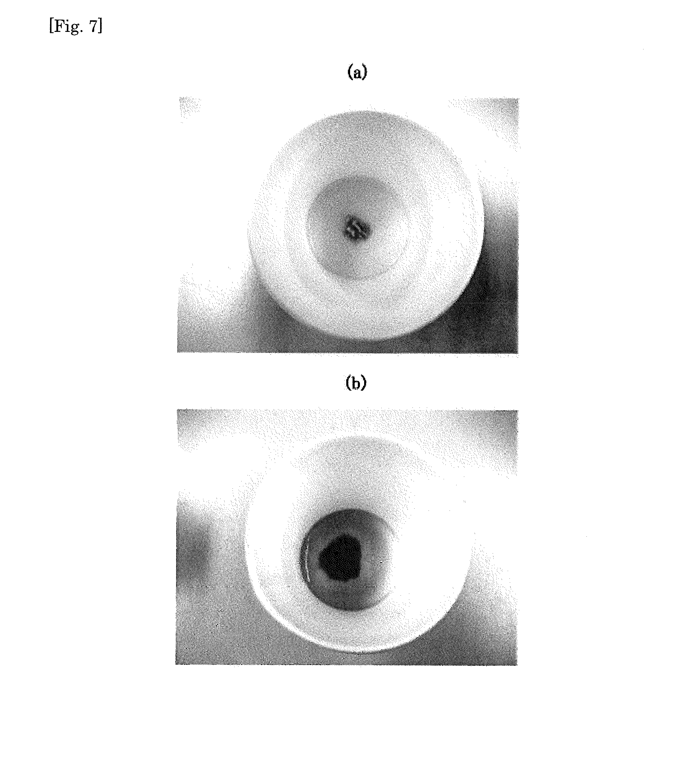

A water-soluble gel was obtained by using gelatin as a substrate of a protease, using phenol red as a pH indicator, putting 4 ml of one obtained by adding phenol red (manufactured by SIGMA-ALDRICH, Product name: Phenol Red, A.C.S reagent) to an aqueous 2% gelatin solution in a paper cup, and then forming a gel. Two pieces of the paper cups containing the gelatin gel were prepared.
As a test substance, 0.05 g of a culture medium in which Dermatophagoides farinae was cultured was used (in which mites already did not survive and which includes bodies and fragments of dead mites, excrements of mites, and mite feed). As a measurement sample with a Der f 1 concentration of 0 ng/ml, 0.05 g of mite feed was used. As the mite feed, a mouse food (MF) and dried yeast were mixed at a weight ratio of 1:1.
The measurement samples was placed in each paper cup containing the gelatin gel previously prepared, and then allowed to stand still at room temperature for 24 hours.
24 hours later, when each paper cup containing the gelatin gel was observed, it was visually confirmed that the gelatin gel decreased and was liquefied only in the paper cup in which the Dermatophagoides farinae culture medium was placed. It was also visually observed from the change in the color of the phenol red that the basicity of the liquefied portion became high (The color change and the pH value correspond to each other.). Fig. 7(a) is a view showing the color change of the gelatin gel after allowed to stand still for 24 hours when only mite feed (dark gray portion at the central portion of the cup) was used as a measurement sample. Fig. 7(b) is a view showing the color change of the gelatin gel after allowed to stand still for 24 hours when the Dermatophagoides farinae culture medium (black portion at the central portion of the cup) was used as a measurement sample.
In Fig. 7(a), the gelatin gel exhibits yellow color which is the original color (in Fig. 7(a), a white portion at the bottom portion of the cup), in which the color change to red was not observed. The dark gray portion at the bottom central portion of the cup shows the mite feed, in which the color change to red was not observed.
On the other hand, Fig. 7(b) shows that a large portion (equal to or wider than the left half portion of the figure) thereof changed the color to red (in Fig. 7(b), the black or dark gray portion at the bottom portion of the cup). The black portion at the bottom central portion of the cup is a portion where the mite culture medium was placed, and the portion changed the color to red.
Thus, the gel-like gelatin was liquefied due to an enzyme reaction of the protease derived from the mite allergen, and the pH which is the physical property of the portion changed. It is considered that the measurement of the allergen amount can be achieved by quantifying or relatively showing the density of the color corresponding to the pH change and the width of the color-changed portion, quantitatively or relatively grasping the allergen amount corresponding thereto, and then quantifying or semi-quantifying the physical property change (pH change) of the water-soluble substrate based on the protease as an index.

### Reference Signs List

1a-1 pH test paper to which (immediately after) a sample with a Der f 1 concentration of 5000 ng/ml was added dropwise
1a-2 pH test paper to which (18 hours after) a sample with a Der f 1 concentration of 0 ng/ml was added dropwise
1a-3 pH test paper to which (18 hours after) a sample with a Der f 1 concentration of 250 ng/ml was added dropwise
1a-4 pH test paper to which (18 hours after) a sample with a Der f 1 concentration of 500 ng/ml was added dropwise
1a-5 pH test paper to which (18 hours after) a sample with a Der f 1 concentration of 1000 ng/ml was added dropwise
1a-6 pH test paper to which (18 hours after) a sample with a Der f 1 concentration of 2500 ng/ml was added dropwise
1a-7 pH test paper to which (18 hours after) a sample with a Der f 1 concentration of 5000 ng/ml was added dropwise
1b-1 pH test paper to which a solution with a pH of 5.0 was added dropwise
1b-2 pH test paper to which a solution with a pH of 6.0 was added dropwise
1b-3 pH test paper to which a solution with a pH of 6.5 was added dropwise
1b-4 pH test paper to which a solution with a pH of 7.0 was added dropwise
1b-5 pH test paper to which a solution with a pH of 7.5 was added dropwise
1b-6 pH test paper to which a solution with a pH of 8.0 was added dropwise
1b-7 pH test paper to which a solution with a pH of 8.2 was added dropwise
1b-8 pH test paper to which a solution with a pH of 8.5 was added dropwise
2a-1 pH test paper to which a measurement sample (2a-1) with a Der f 1 concentration of 5000 ng/ml was added dropwise
2a-2 pH test paper to which a measurement sample (2a-2) with a Der f 1 concentration of 2500 ng/ml was added dropwise
2a-3 pH test paper to which a measurement sample (2a-3) with a Der f 1 concentration of 1000 ng/ml was added dropwise
2a-4 pH test paper to which a measurement sample (2a-4) with a Der f 1 concentration of 500 ng/ml was added dropwise
2a-5 pH test paper to which a measurement sample (2a-5) with a Der f 1 concentration of 250 ng/ml was added dropwise
2a-6 pH test paper to which a measurement sample (2a-6) with a Der f 1 concentration of 0 ng/ml was added dropwise
3a-1 Gelatin gel to which a measurement sample (3a-1) with a Der f 1 concentration of 0 ng/ml was added dropwise
3a-2 Gelatin gel to which a measurement sample (3a-2) with a Der f 1 concentration of 250 ng/ml was added dropwise
3a-3 Gelatin gel to which a measurement sample (3a-3) with a Der f 1 concentration of 500 ng/ml was added dropwise
3a-4 Gelatin gel to which a measurement sample (3a-4) with a Der f 1 concentration of 1000 ng/ml was added dropwise
3a-5 Gelatin gel to which a measurement sample (3a-5) with a Der f 1 concentration of 2500 ng/ml was added dropwise
3a-6 Gelatin gel to which a measurement sample (3a-6) with a Der f 1 concentration of 5000 ng/ml was added dropwise

## Claims

1. A biological allergen measurement method, comprising:
bringing a test substance into contact with a water-soluble gel or aqueous solution containing a substrate of a protease of an environmental biological allergen, and then quantifying or semi-quantifying a physical property change of the water-soluble gel or the aqueous solution caused by an effect of a protease contained in the test substance on the substrate to thereby measure an amount of the environmental biological allergen.

2. The biological allergen measurement method according to Claim 1, wherein the physical property change is liquefaction of the water-soluble gel, and the amount of the environmental biological allergen is measured by quantifying or semi-quantifying a weight or a volume of a portion generated by the liquefaction.

3. The biological allergen measurement method according to Claim 2, wherein the water-soluble gel contains protein which forms a gel at room temperature.

4. The biological allergen measurement method according to Claim 1, wherein the physical property change is a change in a pH of the water-soluble gel or the aqueous solution, and the amount of the environmental biological allergen is measured by quantifying or semi-quantifying the pH change.

5. The biological allergen measurement method according to Claim 4, wherein the water-soluble gel or the aqueous solution contains protein whose pH changes due to an effect of the protease of the allergen.

6. The biological allergen measurement method according to Claim 4 or 5, wherein the water-soluble gel or the aqueous solution contains a pH indicator.

7. The biological allergen measurement method according to any one of Claims 1 to 6, wherein the substrate contains natural product-derived protein and/or protein hydrolyzate.

8. The biological allergen measurement method according to any one of Claims 1 to 7, wherein the test substance is collected from a floor, a wall, a window, a window frame, matting, bedding, furniture, dust particles, and house dust.

9. A simple biological allergen measurement kit for use in the biological allergen measurement method according to any one of Claims 1 to 8,
the simple biological allergen measurement kit comprising the water-soluble gel or the aqueous solution.
